# EUROPÄISCHE PATENTSCHRIFT

(11) **EP 3 008 146 B1**
(45) Veröffentlichungstag und Bekanntmachung des Hinweises auf die Patenterteilung: **05.04.2017**
(21) Anmeldenummer: 14726528.4
(22) Anmeldetag: 23.05.2014
(51) Int. Cl.: C09K 3/18, B01D 1/06, B01D 1/26, C07C 29/74, C07C 29/80

(54) **VERFAHREN ZUR AUFARBEITUNG GLYKOLHALTIGER FLUGZEUGENTEISUNGSMITTEL**
METHOD FOR REPROCESSING AIRCRAFT DE-ICING AGENTS COMPRISING GLYCOL
PROCÉDÉ DE RETRAITEMENT D'AGENTS DE DÉGIVRAGE DES AVIONS CONTENANT DU GLYCOL

(30) Priorität: 13.06.2013 DE 102013009949
(43) Veröffentlichungstag der Anmeldung: 20.04.2016
(73) Patentinhaber: Clariant International Ltd, 4132 Muttenz (CH)
(72) Erfinder: STANKOWIAK, Achim, 84503 Altötting (DE)
(74) Vertreter: Mikulecky, Klaus
(86) Internationale Anmeldenummer: PCT/EP2014/001413
(87) Internationale Veröffentlichungsnummer: WO 2014/198379

(56) Entgegenhaltungen:
- EP-A2- 1 018 500
- WO-A1-92/04956
- WO-A2-2010/072312
- US-A- 5 535 877

## Beschreibung

Die Erfindung betrifft ein Verfahren zur Wiederaufbereitung von gebrauchten Flugzeugenteisungsmitteln auf der Basis von Glykolen.

Flugzeugenteisungsmittel auf Basis von Glykolen sind zum Beispiel in US-4 358 389 und US-4 744 913 beschrieben. Sie enthalten im Allgemeinen
(a) etwa 40 bis 80 Gew.-% mindestens eines Glykols mit 2 oder 3 Kohlenstoffatomen oder eines Diglykols mit 4 bis 6 Kohlenstoffatomen, zum Beispiel Ethylenglykol, Diethylenglykol, Propylenglykol und dergleichen,
(b) 0,05 bis 1,5 Gew.-% mindestens einer polymeren Komponente als Verdickungsmittel, zum Beispiel aus der Gruppe der Polyacrylate, Polymethacrylate, Xanthangummi und Cellulosederivate,
(c) 0,05 bis 1 Gew.-% mindestens eines Tensids, zum Beispiel Olefinsulfonate, Alkylarylsulfonate, Polyoxalkylate und dergleichen,
(d) mindestens einen Korrosionsinhibitor in einer wirksamen Menge, zum Beispiel aus der Gruppe der Triazole, Imidazole und/oder Phosphorsäureester, und
(e) mindestens eine basischen Verbindung zur Einstellung des pH-Wertes von etwa 7,5 bis 11 und
(f) Wasser als Rest auf 100 Gew.-%.

Diese Flugzeugenteisungsmittel werden als solche (das heißt als Konzentrat) oder nach Verdünnung mit Wasser zu deren Konservierung und/oder zur Befreiung von Eis und/oder Schnee auf die zu behandelnden Flugzeugteile aufgebracht. Von den behandelten Flugzeugteilen fließt das Enteisungsmittel, das nun mit Schmelzwasser mehr oder weniger verdünnt und mit Sand, Gummiabrieb, Öl, Verbrennungsrückständen und dergleichen verunreinigt ist, in ein Sammelbecken und wird als Abwasser aus der Flugzeugenteisung oder als gebrauchtes Flugzeugenteisungsmittel bezeichnet.

Teilweise werden die gebrauchten Flugzeugenteisungsmittel mit Hilfe einer biologischen Kläranlage entsorgt. Dies führt aber - trotz der guten biologischen Abbaubarkeit von Glykolen - zu einer unerwünschten Belastung der Kläranlage, besonders bei niedrigen Außentemperaturen und damit verbundener reduzierter Bakterienaktivität, was beim Einsatz von Enteisungsmitteln im Allgemeinen der Fall ist. Ein weiterer Nachteil dieser Art der Entsorgung von Flugzeugenteisungsmitteln ist der Verlust des in großer Menge vorliegenden und wertvollen Glykols.

EP-A-0 637 620 offenbart ein Verfahren zur Wiederaufbereitung von gebrauchten Flugzeugenteisungsmitteln auf der Basis von Glykolen, in dem man
(1) das gebrauchte Flugzeugenteisungsmittel zunächst zur Abtrennung von den suspendierten Verunreinigungen filtriert,
(2) das im Schritt (1) erhaltene Filtrat zur Abtrennung von den polymeren Verdickungsmitteln einer Ultrafiltration unterwirft,
(3) das im Schritt (2) erhaltene Permeat zur Abtrennung von anwesenden Salzen und ionischen Verbindungen der Ultrafiltration mit einem Anionenaustauscher und einem Kationenaustauscher unterwirft, und
(4) die im Schritt (3) erhaltene Lösung zur Entfernung von überschüssigem Wasser und damit Einstellung des Glykolgehaltes auf den gewünschten Wert destilliert.
(5) das erhaltene Glykol-Wassergemisch mit geeigneten Additiven für den Einsatz als Flugzeugenteisungsmittel nachadditiviert.

EP-A-1 889 658 offenbart ein Verfahren zur Aufarbeitung glykolhaltiger Flugzeugenteisungsmittel, bei dem diese unmittelbar einer Trennung mittels einer Membran unterworfen werden, wobei sich in einem späteren Verfahrensschritt eine Destillation ausschließt. Dieses anlagentechnisch anspruchsvolle und aufwändige Verfahren zur Wiederverwendung gebrauchter Flugzeugenteisungsmittel macht nur an Flughäfen Sinn, die einen großen Bedarf an Flugzeugenteisungsmittel haben.

EP 2 382 279 offenbart ein Verfahren zur Aufarbeitung glykolhaltiger Flugzeugenteisungsmittel, in dem man
(1) das gebrauchte und gegebenenfalls mit Landebahnenteisungsmittel verunreinigte Flugzeugenteisungsmittel in einer geeigneten Vorrichtung sammelt,
(2) das gebrauchte Flugzeugenteisungsmittel nachfolgend ohne, oder nach nur grober vorheriger Abtrennung fester oder suspendierter Verunreinigungen, durch Austreiben von Wasser bei erhöhter Temperatur auf einen Glykolgehalt zwischen 55 und 75 Gew.-% bringt,
(3) das so erhaltene, aufkonzentrierte gebrauchte Flugzeugenteisungsmittel zu einer zentralen Aufbereitungsanlage transportiert, wo jenes einer Feindestillation unterzogen wird, und wobei
(4) das Glykol als Destillat der Feindestillation anfällt.

EP 1 018 500 offenbart ein Verfahren zur Isolierung von Glykolen aus einem Glykole, Wasser und Salze enthaltenden flüssigen Gemisch durch (a) Verdampfung zumindest einer Teilmenge des Wassers und der Glykole, die im Gemisch enthalten sind, unter Abscheidung eines von Salz befreiten, Glykole und Wasser aufweisenden, flüssigen oder gasförmigen Mediums, (b) Entwässerung des Mediums und (c) Isolierung der Glykole aus dem entwässerten Medium, dadurch gekennzeichnet, dass zur Durchführung der Stufe (a) ein Dünnschichtverdampfer eingesetzt wird.

WO 92/04956 beschreibt ein Verfahren in dem aus ggf. filtrierten und von an der Oberfläche abgeschiedenem Öl befreiten Altkühlwasser A, z. B bestehend aus etwa 57 % Wasser, 38 % Ethylenglykol und 5 % Nicht- bzw. Schwersiedern, zunächst durch eine erste Verdampfungsstufe, die aus einem Verdampfer 1 besteht, der vorzugsweise als Dünnschichtverdampfer ausgelegt ist, die Hauptmenge des Wassers, vorzugsweise unter Atmosphärendruck, destillativ abgetrennt wird.

Es wurde nun beobachtet, dass das nach dem Stand der Technik wieder gewonnene und in Enteisungsmittel eingearbeitete Glykol eine nur ungenügende pH-Stabilität aufweist. Es enthält einen Anteil von Glykolestern und anderen Verunreinigungen, der die weitere Verwendung als Flugzeugenteisungsmittel ausschließt, erheblich erschwert oder nur eingeschränkt zulässt. Die Aufgabe der vorliegenden Erfindung besteht darin, ein einfaches und kostengünstiges Verfahren vorzuschlagen, das ein weitgehendes Wiedergewinnen und Wiedereinsetzen insbesondere der Glykole von gebrauchten Flugzeugenteisungsmitteln ermöglicht, und wobei die Wiederverwendbarkeit der Glykole erhalten bleibt. Das Verfahren soll mit geringerem anlagentechnischem Aufwand durchführbar sein, so dass ein Betrieb einer entsprechenden Anlage an einem Flughafen möglich ist. Das Verfahren soll ein aufgearbeitetes Produkt mit möglichst wenigen Nebenprodukten ergeben, so dass seine Weiterverarbeitung zu neuem Flugzeugenteisungsmittel dessen Lebensdauer nicht beeinträchtigt. Ferner soll der Aufwand für Energie möglichst gering sein. Gegenstand vorliegender Erfindung ist somit ein Verfahren zur Aufarbeitung glykolhaltiger Flugzeugenteisungsmittel, in dem man
(1) das gebrauchte und gegebenenfalls mit Landebahnenteisungsmittel verunreinigte Flugzeugenteisungsmittel in einer geeigneten Vorrichtung sammelt,
(2) das so gesammelte gebrauchte Flugzeugenteisungsmittel nachfolgend ohne, oder nach nur grober vorheriger Abtrennung fester oder suspendierter Verunreinigungen, durch Austreiben von Wasser auf einen Glykolgehalt zwischen 50 und 75 Gew.-% bringt,
(3) und das so gewonnene Konzentrat mittels eines Dünnschichtverdampfers in ein Wasser/Glykolgemisch und einen Rückstand trennt, und
(4) das Wasser/Glykolgemisch sammelt.

In Schritt 2 wird Wasser in einem Umfang ausgetrieben, so dass das daraus erhaltene Konzentrat einen Glykolgehalt von vorzugsweise 55 bis 70 Gew.-% aufweist. Das Austreiben des Wassers erfolgt vorzugsweise bei erhöhter Temperatur. Wasser kann aber auch durch Reduzierung des Drucks abgezogen werden, oder durch Reduzierung des Drucks und erhöhte Temperatur.

In Schritt 3 wird das aus dem gebrauchten Flugzeugenteisungsmittel gewonnene Konzentrat zur Rückgewinnung eines gereinigten Wasser-Glykofgemischs dem Dünnschichtverdampfer zugeführt. Das Wasser-Glykolgemisch wird als Destillat des Dünnschichtverdampfers erhalten. Der Rückstand, der den Dünnschichtverdampfer verlässt, wird in einer Ausführungsform der Erfindung verworfen. In einer anderen Ausführungsform wird der Rückstand erneut einem Dünnschichtverdampfer zugeführt, und das so erhaltene Destillat kann mit dem beim ersten Durchgang erhaltenen Destillat vereinigt werden.

Die Betriebsbedingungen des Dünnschichtverdampfers sind so zu wählen, dass das entstehende Wasser/Glykolgemisch einen Glykolgehalt von vorzugsweise 30 bis 90 Gew.-%, insbesondere 45 bis 80 Gew.-%, speziell 45 - 65 Gew.-% beispielsweise 45 - 60 Gew.-% Glykol aufweist.

Bei den Glykolen handelt es sich in bevorzugter Ausführungsform um Glykole mit 2 oder 3 Kohlenstoffatomen oder Diglykole mit 4 bis 6 Kohlenstoffatomen, zum Beispiel Ethylenglykol, Diethylenglykol oder Propylenglykol. Besonders bevorzugt sind Propylenglykol und Monoethylenglykol, insbesondere Propylenglykol. Zwischen den Schritten 2 und 3, also Abziehen des Wassers und Dünnschichtverdampfung, kann das aus Schritt 2 erhaltene Zwischenprodukt mit wässriger Lauge, vorzugsweise mit Natron- oder Kalilauge, auf einen alkalischen pH eingestellt werden.

Das gebrauchte Flugzeugenteisungsmittel wird von der Stelle, an der die Flugzeuge auf dem Flughafengelände enteist werden in ein geeignetes Behältnis wie einen Lagertank oder ein Auffangbecken geleitet. Vor dem Eintritt in den Lagertank oder ein Auffangbecken kann das gebrauchte Flugzeugenteisungsmittel filtriert werden, um grobe Verunreinigungen zu entfernen.

Von dort wird das gebrauchte Flugzeugenteisungsmittel in eine geeignete Vorrichtung verbracht, in der, vorzugsweise durch Erhitzen, das Wasser teilweise ausgetrieben werden kann. Hierbei ist die Einhaltung von CSB-Grenzen im Kondensat, abhängig von den lokalen Vorschriften, zu berücksichtigen. Der Glykolgehalt des Konzentrats aus der Wasseraustreibung liegt zwischen 50 und 75 Gew.-% Glykol. Anschließend wird das so gewonnene Konzentrat dem Dünnschichtverdampfer zugeführt. Die Betriebsbedingungen des Dünnschichtverdampfers sind beispielsweise:

| | |
|---|---|
| Manteltemperatur: | 140 - 160 °C |
| Kopftemperatur: | 50 - 104 °C |
| Druck: | 80 - 120 mbar |
| Kopf Sumpfverhältnis: | ca. 1:1 bis 2:1 |

Das aus dem Dünnschichtverdampfer erhaltene Destillat erfordert keine weitere Aufarbeitung. Es kann unmittelbar durch die Zufügung von geeigneten Zusatzstoffen zu neuem Flugzeugenteisungsmittel weiter verarbeitet werden. Es kann erforderlich sein, den Glykolgehalt entsprechend der Spezifikation durch Zugabe von Wasser oder Glykol einzustellen.

Das erfindungsgemäße Verfahren ist besonders geeignet für Flugenteisungsmittel, die den international gültigen Normen und Spezifikationen, wie sie in AMS 1424 und AMS 1428 festgelegt sind.

Solche Flugzeugenteisungsmittel enthalten, neben Wasser, beispielsweise:
(a) 50 bis 95 Gew.-%, Glykole mit 2 oder 3 Kohlenstoffatomen oder Diglykole mit 4 bis 6 Kohlenstoffatomen,
(b) 0 bis zu 0,8 Gew.-%, vorzugsweise 0 bis 0,5 Gew.-%, wasserlösliche Polymere aus der Gruppe der Polyacrylate und Polymethacrylate als Verdicker,
(c) 0,01 bis 1 Gew.-% Tenside, vorzugsweise aus der Gruppe der anionischen Tenside, zum Beispiel Sulfonate wie Olefinsulfonate und Alkylbenzolsulfonate,
(d) 0,001 bis 0,1 Gew.-% Korrosionsinhibitor, z.B. Salze der Phosphor- und Bernsteinsäure

Die angegebene Zusammensetzung bezieht sich auf das Flugzeugenteisungsmittel vor seinem Gebrauch.

Die angestrebte Qualität der mit dem erfindungsgemäßen Verfahren gewonnenen Glykole wird durch folgende Kriterien festgelegt:
(a) eine Tensidkonzentration von weniger als 100 ppm
(b) einen Glykolgehalt von 30 - 90 Gew.-% vorzugsweise 45 - 80 Gew.-%, speziell 45 - 60 Gew.-%
(c) ein Glykolsäuregehalt von weniger als 100 ppm, vorzugsweise weniger als 50 ppm
(d) ein Ameisensäuregehalt von weniger als 100 ppm, vorzugsweise weniger als 50 ppm
(e) ein Milchsäuregehalt von weniger als 100 ppm, vorzugsweise weniger als 50 ppm
(f) ein Essigsäuregehalt von weniger als 100 ppm, vorzugsweise weniger als 50 ppm
(g) ein Propionsäuregehalt von weniger als 100 ppm, vorzugsweise weniger als 50 ppm

Der angestrebte Gehalt an Glykolestern der unter (c) bis (g) genannten Säuren ist höchstens so groß wie der Gehalt an den Säuren.

Das erfindungsgemäße Verfahren bietet gegenüber dem Stand der Technik erhebliche Vorteile.
1. Es wird eine Destillationskolonne weniger benötigt als bei destillativen Aufarbeitungsverfahren. Diese ist ein wesentlicher Teil einer Investition in eine Recyclinganlage, welche direkt am Flughafen oder in dessen Nähe installiert werden soll.
2. Das aus dem Dünnschichtverdampfer erhaltene Produkt (mit 30 bis 90 Gew.-% Glykolgehalt) wurde thermisch wesentlich schonender behandelt als bei destillativen Aufarbeitungsverfahren, es wurde nur kurz im Dünnschichtverdampfer erhöhten Temperaturen ausgesetzt. Als Folge davon ist der Gehalt an Nebenprodukten im so erhaltenen Wasser/Glykolgemisch sehr gering.
3. Nach dem Verfahren des Standes der Technik destilliert man zuerst alles Wasser ab, um es dann nach der Feindestillation wieder in dem Produktionsprozess einzusetzen. Das erfindungsgemäße Verfahren führt sofort zu einem verwendbaren Wasser/Glykolgemisch, was in erheblichem Umfang Energie einspart.

### Beispiel 1

Aufkonzentriertes, gebrauchtes Flugzeugenteisungsmittel auf Basis von Propylenglykol mit einem Wassergehalt von 43 % (der Gehalt an Propylenglykol lag daher zwischen 56 und 57 Gew.-%) wurde mittels 45 %iger wässriger KOH auf einen alkalischen pH eingestellt und einer Dünnschichtverdampfung unterworfen.

Betriebsbedingungen des Dünnschichtverdampfers:

| | |
|---|---|
| Manteltemperatur: | 150 °C |
| Kopftemperatur: | 55-76 °C |
| Druck: | 100 mbar |
| Kopf Sumpfverhältnis: | ca. 1:1 bis 2:1 |

Der Sumpf wurde anschließend erneut einer Dünnschichtverdampfung unterworfen.

Betriebsbedingungen des Dünnschichtverdampfers:

| | |
|---|---|
| Manteltemperatur: | 150 °C |
| Kopftemperatur: | 90 - 104 °C |
| Druck: | 100 mbar |
| Kopf Sumpfverhältnis: | ca. 2:1 |

Der Propylenglykolgehalt der vereinigten Destillate betrug ca. 47 %

### Beispiel 2

Aufkonzentriertes, gebrauchtes Flugzeugenteisungsmittel auf Basis von Propylenglykol mit einem Wassergehalt von 43 % (der Gehalt an Propylenglykol lag daher zwischen 56 und 57 Gew.-%) wurde mittels 45%iger wässriger KOH auf einen alkalischen pH eingestellt und einer Dünnschichtverdampfung unterworfen. Betriebsbedingungen des Dünnschichtverdampfers:

| | |
|---|---|
| Manteltemperatur: | 150 °C |
| Kopftemperatur: | 80 - 90 °C |
| Druck: | 100 mbar |
| Kopf Sumpfverhältnis: | ca. 2:1 |

Der Sumpf wurde anschließend erneut einer Dünnschichtverdampfung unterworfen.

Betriebsbedingungen des Dünnschichtverdampfers:

| | |
|---|---|
| Manteltemperatur: | 150 °C |
| Kopftemperatur: | 80 - 98 °C |
| Druck: | 100 mbar |
| Kopf Sumpfverhältnis: | ca. 2:1 |

Der Propylenglykolgehalt der vereinigten Destillate betrug ca. 49 %. Die folgende Tabelle zeigt die Eigenschaften des aus der Dünnschichtverdampfung erhaltenen Produkts.

| Beispiel | | 1 | 2 |
|---|---|---|---|
| Wassergehalt (DIN 51777) | [%] | 53,6 | 51 |
| Tensidgehalt | [ppm] | <50 | <50 |
| Glykolsäure | [ppm] | <20 | <20 |
| Ameisensäure | [ppm] | <20 | <40 |
| Milchsäure | [ppm] | <40 | <20 |
| Essigsäure | [ppm] | <20 | <20 |
| Propionsäure | [ppm] | <20 | <20 |

## Patentansprüche

1. Verfahren zur Aufarbeitung glykolhaltiger Flugzeugenteisungsmittel, in dem man
(1) das gebrauchte und gegebenenfalls mit Landebahnenteisungsmittel verunreinigte Flugzeugenteisungsmittel in einer geeigneten Vorrichtung sammelt,
(2) das so gesammelte gebrauchte Flugzeugenteisungsmittel nachfolgend ohne, oder nach nur grober vorheriger Abtrennung fester oder suspendierter Verunreinigungen, durch Austreiben von Wasser auf einen Glykolgehalt zwischen 50 und 75 Gew.-% bringt,
(3) und das so gewonnene Konzentrat mittels eines Dünnschichtverdampfers in ein Wasser/Glykolgemisch und einen Rückstand trennt, und
(4) das Wasser/Glykolgemisch sammelt.

2. Verfahren nach Anspruch 1, in dem die Abtrennung fester oder suspendierter Verunreinigungen in Schritt (2) eine Grobfiltration umfasst.

3. Verfahren nach Anspruch 1 und/oder 2, in dem das Flugzeugenteisungsmittel vor seinem Gebrauch neben Wasser
(a) 50 bis 95 Gew.-% Glykole mit 2 oder 3 Kohlenstoffatomen oder Diglykole mit 4 bis 6 Kohlenstoffatomen,
(b) 0 bis zu 0,8 Gew.-% wasserlösliche Polymere aus der Gruppe der Polyacrylate und Polymethacrylate,
(c) 0,01 bis 1 Gew.-% Tenside,
(d) 0,001 bis 1 Gew.-% Korrosionsinhibitor enthält.

4. Verfahren nach einem oder mehreren der Ansprüche 1 bis 3, wobei die Betriebsbedingungen des Dünnschichtverdampfers so gewählt sind, dass das erhaltene Wasser/Glykolgemisch einen Glykolgehalt von von 30 - 90 Gew.-% vorzugsweise 45 - 65 Gew.-% aufweist.

5. Verfahren nach einem oder mehreren der Ansprüche 1 bis 4, wobei das Glykol Propylenglykol oder Monoethylenglykol ist.

6. Verfahren nach einem oder mehreren der Ansprüche 1 bis 5, wobei zwischen Schritt 2 und Schritt 3 das aus Schritt 2 erhaltene Zwischenprodukt mittels einer wässrigen Lauge auf einen alkalischen pH-Wert eingestellt wird.

## Claims

1. A method for reprocessing aircraft de-icing agents comprising glycol, in which
(1) the used aircraft de-icing agent, possibly contaminated with runway de-icing agent, is collected in a suitable device,
(2) the thus collected used aircraft de-icing agent is subsequently brought to a glycol content between 50 and 75% by weight without, or after only coarse, prior separation off of solid or suspended impurities, by expelling water,
(3) and the thus obtained concentrate is separated by means of a thin-film evaporator into a water/glycol mixture and a residue, and
(4) the water/glycol mixture is collected.

2. The method as claimed in claim 1, in which the separation off of solid or suspended impurities in step (2) involves a coarse filtration.

3. The method as claimed in claim 1 and/or 2, in which the aircraft de-icing agent comprises, prior to its use, besides water,
(a) 50 to 95% by weight of glycols having 2 or 3 carbon atoms or diglycols having 4 to 6 carbon atoms,
(b) 0 up to 0.8% by weight of water-soluble polymers from the group of polyacrylates and polymethacrylates,
(c) 0.01 to 1% by weight of surfactants,
(d) 0.001 to 1% by weight of corrosion inhibitor.

4. The method as claimed in one or more of claims 1 to 3, where the operating conditions of the thin-film evaporator are selected such that the resulting water/glycol mixture has a glycol content of from 30-90% by weight, preferably 45-65% by weight.

5. The method as claimed in one or more of claims 1 to 4, where the glycol is propylene glycol or monoethylene glycol.

6. The method as claimed in one or more of claims 1 to 5, where, between step 2 and step 3, the intermediate product obtained from step 2 is adjusted to an alkaline pH using an aqueous hydroxide solution.

## Revendications

1. Procédé pour le traitement d'un produit de dégivrage contenant du glycol pour avions, dans lequel
(1) on rassemble le produit de dégivrage pour avion usagé et le cas échéant contaminé par du produit de dégivrage de piste d'atterrissage dans un dispositif approprié,
(2) on amène ensuite le produit de dégivrage pour avion usagé ainsi rassemblé, sans séparation ou après une séparation préalable uniquement grossière de contaminations solides ou en suspension, par évacuation d'eau, à une teneur en glycol entre 50 et 75% en poids,
(3) et on sépare le concentrat ainsi obtenu au moyen d'un évaporateur à couche mince en un mélange eau/glycol et en un résidu et
(4) on rassemble le mélange eau/glycol.

2. Procédé selon la revendication 1, dans lequel la séparation de contaminations solides ou en suspension dans l'étape (2) comprend une filtration grossière.

3. Procédé selon la revendication 1 et/ou 2, dans lequel l'agent de dégivrage pour avions contient, avant son utilisation, outre de l'eau
(a) 50 à 95% en poids de glycols comprenant 2 à 3 atomes de carbone ou de diglycols comprenant 4 à 6 atomes de carbone,
(b) 0 à jusqu'à 0,8% en poids de polymères solubles dans l'eau du groupe des polyacrylates et des polyméthacrylates,
(c) 0,01 à 1% en poids de tensioactifs,
(d) 0,001 à 1% en poids d'inhibiteur de corrosion.

4. Procédé selon l'une ou plusieurs des revendications 1 à 3, les conditions de fonctionnement de l'évaporateur à couche mince étant choisies de manière telle que le mélange eau/alcool obtenu présente une teneur en glycol de 30-90% en poids, de préférence de 45-65% en poids.

5. Procédé selon l'une ou plusieurs des revendications 1 à 4, le glycol étant le propylèneglycol ou le monoéthylèneglycol.

6. Procédé selon l'une ou plusieurs des revendications 1 à 5, le produit intermédiaire de l'étape 2, obtenu entre l'étape 2 et l'étape 3, étant réglé à un pH alcalin au moyen d'une lessive aqueuse.
